# EUROPEAN PATENT APPLICATION

(11) **EP 2 495 246 A1**
(43) Date of publication of application: **05.09.2012**
(21) Application number: 10826667.7
(22) Date of filing: 25.10.2010
(51) Int. Cl.: C07D 487/02

(54) **FULLERENE DERIVATIVE**

(30) Priority: 30.10.2009 JP 2009249936
(71) Applicant: Sumitomo Chemical Company, Limited, Tokyo 104-8260 (JP); National University Corporation Tottori University, Tottori-shi Tottori 680-8550 (JP)
(72) Inventor: ITO, Toshiyuki, Tottori-shi Tottori 680-0834 (JP); UETANI, Yasunori, Tsukuba-shi Ibaraki 305-0045 (JP)
(74) Representative: Weinberger, Rudolf
(86) International application number: PCT/JP2010/068843
(87) International publication number: WO 2011/052535

(57) **Abstract**

A fullerene derivative having two or more structures represented by the formula (1) can increase light absorption in a long wavelength range when used for an organic photovoltaic cell. 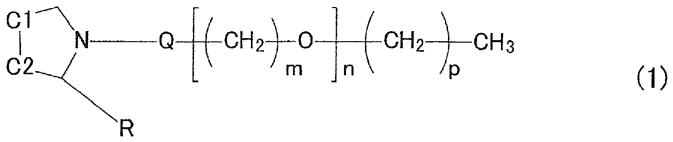 In the formula (1), C1 and C2 represent carbon atoms constituting the fullerene skeleton. m represents an integer from 1 to 6, and n represents an integer from 1 to 4. p represents an integer of 0 or more, and R represents a monovalent organic group. Q represents a single bond, an arylene group, or a divalent heterocyclic group. When m is plurally present, these m may be the same or different from each other.

## Description

### TECHNICAL FIELD

The present invention relates to a fullerene derivatives and to an organic photovoltaic cell.

### BACKGROUND ART

Organic semiconductor materials have the ability to transport charges being electrons and holes, and it is contemplated to use such organic semiconductor materials for, for example, organic photovoltaic cells such as organic solar cells and optical sensors. For example, organic solar cells using fullerene derivatives are being studied.

One known example of such fullerene derivatives is N-hexadecyl-2-(2-naphthyl)fulleropyrrolidine obtained by adding one functional group to a fullerene having 60 carbon atoms (this may be referred to as a C₆₀ fullerene. A fullerene having n carbon atoms may be hereinafter referred to as a Cₙ fullerene) (see Patent Document 1).
Another known example is a fullerene derivative referred to as bis-[60]PCBM obtained by adding a plurality of functional groups to a C₆₀ fullerene (see Patent Document 2).

### RELATED ART DOCUMENTS

### Patent Document

Patent Document 1: JP 2009-84264 A
Patent Document 2: WO 2009/039490 A

### DISCLOSURE OF INVENTION

However, organic photovoltaic cells using conventional fullerene derivatives do not sufficiently absorb light in a long wavelength range of 500 nm or longer. Therefore, the photovoltaic efficiency of the organic photovoltaic cells using the conventional fullerene derivatives may be low.

The present invention provides novel fullerene derivatives having a certain structure. The use of the fullerene derivatives for an organic photovoltaic cell allows an increase in light absorption in the long wavelength range. The invention also provides an organic photovoltaic cell having excellent photovoltaic efficiency.

More specifically, the present invention provides the following fullerene derivatives and organic photovoltaic cells.
[1] A fullerene derivative comprising two or more structures represented by the following formula (1): 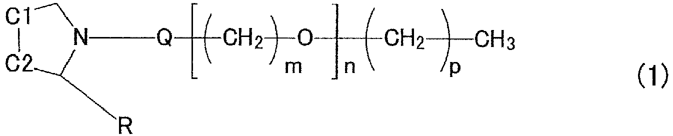 wherein, in the formula (1), C1 and C2 represent carbon atoms constituting a fullerene skeleton, m represents an integer from 1 to 6, n represents an integer from 1 to 4, p represents an integer 0 or more, R represents a monovalent organic group, and Q represents a single bond, an arylene group, or a divalent heterocyclic group, and wherein when m is plurally present, these m may be the same or different from each other.
[2] The fullerene derivative according to above [1], represented by the following formula (2): 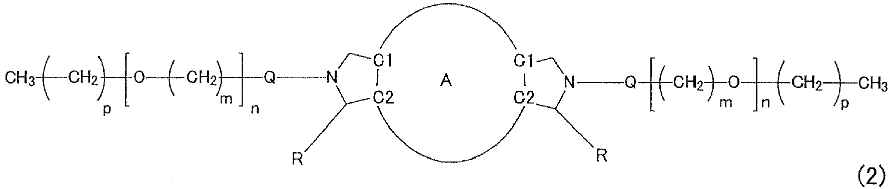 wherein, in the formula (2), an A ring represents a fullerene skeleton having 60 or more carbon atoms, and the definitions of C1, C2, m, n, p, Q, and R are the same as the definitions for the formula (1), and wherein two of Q may be the same or different from each other, two of R may be the same or different from each other, a plurality of m may be the same or different from each other, two of n may be the same or different from each other, and two of p may be the same or different from each other.
[3] The fullerene derivative according to above [1] or [2], wherein R is a group represented by the following formula (3) : 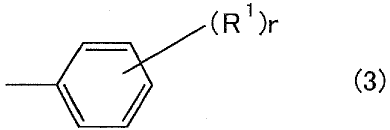 wherein, in the formula (3), R¹ represents an alkyl group, an alkoxy group, an aryl group, a monovalent heterocyclic group, or a halogen group, and r represents an integer from 0 to 5, and wherein when R¹ is plurally present, these R¹ may be the same or different from each other.
[4] The fullerene derivative according to above [3], wherein the A ring is a fullerene skeleton having 60 or more carbon atoms, R¹ is a methoxy group, m is 2, n is 2, p is 0, Q is a single bond, and r is 1.
[5] A composition comprising the fullerene derivative according to any one of above [1] to [4] and an electron donor compound.
[6] The composition according to above [5], wherein the electron donor compound is a macromolecular compound.
[7] An organic photovoltaic cell comprising:
   a pair of electrodes comprising an anode and a cathode; and
   an active layer placed between the pair of electrodes and comprising the fullerene derivative according to any one of above [1] to [4].
[8] An organic photovoltaic cell comprising:
   a pair of electrodes comprising an anode and a cathode; and
   an active layer placed between the pair of electrodes and comprising the composition according to above [5].
[9] An organic photovoltaic cell comprising:
   a pair of electrodes comprising an anode and a cathode; and
   an active layer placed between the pair of electrodes, the active layer comprising an electron acceptor layer comprising the fullerene derivative according to any one of above [1] to [4] and an electron donor layer comprising an electron donor compound, the electron donor layer being joined to the electron donor layer.

### BRIEF DESCRIPTION OF DRAWINGS

FIG. 1 is a schematic cross-sectional view illustrating a configuration example (1) of an organic photovoltaic cell.
FIG. 2 is a schematic cross-sectional view illustrating a configuration example (2) of the organic photovoltaic cell.
FIG. 3 shows the MALDI-TOF MS spectrum of a fullerene derivative A synthesized in Example 1.
FIG. 4 is a graph showing the absorption spectrum of thin films each comprising a fullerene derivative and poly-3-hexylthiophene (which may be referred to as P3HT).

### EXPLANATIONS OF LETTERS OR NUMERALS

- 10: organic photovoltaic cell
- 20: substrate
- 32: first electrode
- 34: second electrode
- 40: active layer
- 42: electron donor layer
- 44: electron acceptor layer

### DESCRIPTION OF EMBODIMENTS

The present invention will be described in detail. In the following description, the drawings may be referred to for explanation. However, the drawings only schematically show the shape, size, and arrangement of each component so that the invention can be understood, and the invention is not particularly limited thereto. The same components in the drawings are denoted by the same reference numerals, and a redundant description may be omitted.

### <Fullerene derivatives>

Fullerene derivatives of the present invention has two or more structures represented by the following formula (1).

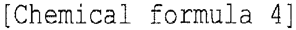

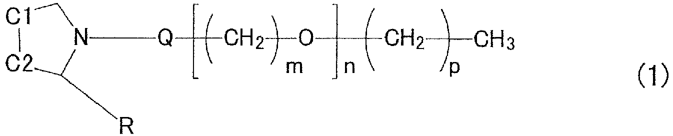

In the structure of the formula (1), C1 and C2 represent carbon atoms constituting the fullerene skeleton.
In the structure of the formula (1), R represents a monovalent organic group. The monovalent organic group is preferably an alkyl group, an alkoxy group, an aryl group, a halogen group, or a monovalent heterocyclic group.

In the structure of the formula (1), when R is an alkyl group, the number of carbon atoms in the alkyl group is generally 1 to 20, and the alkyl group may be a linear or branched alkyl group or may be a cycloalkyl group. Specific examples of the alkyl group may include a methyl group, an ethyl group, a propyl group, an isopropyl group, a butyl group, an isobutyl group, a tert-butyl group, a sec-butyl group, a 3-methylbutyl group, a n-pentyl group, a n-hexyl group, a 2-ethylhexyl group, a n-heptyl group, a n-octyl group, a n-nonyl group, a n-decyl group, and a n-lauryl group. A hydrogen atom in the alkyl group may be substituted with a halogen atom, and such the alkyl group may be a monohalomethyl group, a dihalomethyl group, a trihalomethyl group, or a pentahaloethyl group. The halogen atom is preferably a fluorine atom. Specific examples of the alkyl group comprising a fluorine atom substituted for a hydrogen atom may include a trifluoromethyl group, a pentafluoroethyl group, a perfluorobutyl group, a perfluorohexyl group, and a perfluorooctyl group.

In the structure of the formula (1), when R is an alkoxy group, the number of carbon atoms in the alkoxy group is generally 1 to 20, and the alkoxy group may be a linear or branched alkoxy group or may be a cycloalkyloxy group. Specific examples of the alkoxy group may include a methoxy group, an ethoxy group, a propyloxy group, an iso-propyloxy group, a butoxy group, an iso-butoxy group, a sec-butoxy group, a tert-butoxy group, a n-pentyloxy group, a n-hexyloxy group, a cyclohexyloxy group, a n-heptyloxy group, a n-octyloxy group, a 2-ethylhexyloxy group, a n-nonyloxy group, a n-decyloxy group, a 3,7-dimethyloctyloxy group, and a n-lauryloxy group. A hydrogen atom in the alkoxy group may be substituted with a halogen atom. The halogen atom is preferably a fluorine atom. Specific examples of the alkoxy group comprising a fluorine atom substituted for a hydrogen atom may include a trifluoromethoxy group, a pentafluoroethoxy group, a perfluorobutoxy group, a perfluorohexyloxy group, and a perfluorooctyloxy group.

In the structure of the formula (1), when R is an aryl group, the number of carbon atoms in the aryl group is generally 6 to 60, and the aryl group may have a substituent. Specific examples of the aryl group may include a phenyl group, C₁ to C₁₂ alkoxyphenyl groups ("C₁ to C₁₂" means that the number of carbon atoms is 1 to 12. The same applies to the following), C₁ to C₁₂ alkylphenyl groups, a 1-naphthyl group, and a 2-naphthyl group. Aryl groups having 6 to 20 carbon atoms are preferred, and C₁ to C₁₂ alkoxyphenyl groups and C₁ to C₁₂ alkylphenyl groups are more preferred. A hydrogen atom in the aryl group may be substituted with a halogen atom. The halogen atom is preferably a fluorine atom. Examples of the substituent optionally contained in the aryl group may include linear and branched alkyl groups having 1 to 20 carbon atoms, cycloalkyl groups having 3 to 20 carbon atoms, and alkoxy groups comprising, in their structures, a linear or branched alkyl group having 1 to 20 carbon atoms or a cycloalkyl group having 3 to 20 carbon atoms.

In the structure of the formula (1), when R is a halogen group, the halogen group may be a fluoro group, a chloro group, a bromo group, or an iode group.

In the structure of the formula (1), when R is a monovalent heterocyclic group, the monovalent heterocyclic group is preferably a monovalent aromatic heterocyclic group. The monovalent heterocyclic group may be a thienyl group, a pyridyl group, a furyl group, a piperidyl group, a quinolyl group, an isoquinolyl group, and a pyrrolyl group.

In the structure of the formula (1), m represents an integer from 1 to 6, n represent an integer from 1 to 4, and p represents an integer from 0 to 5. When m is plurally present in the structure of the formula (1), the values of the m may be the same or different from each other. When n is plurally present, the values of the n may be the same or different from each other. When the p is plurally present, the values of the p may be the same or different from each other.

In the structure of the formula (1), it is preferable, from the viewpoint of availability of raw materials, that m is 2. From the viewpoint of charge transportability, p is preferably an integer from 0 to 5 and more preferably an integer from 0 to 3.

In the structure of the formula (1), Q represents a single bond, an arylene group, or a divalent heterocyclic group.

When Q is an arylene group, the number of carbon atoms in the arylene group is generally about 6 to about 60. Specific examples may include a phenylene group, a biphenylene group, a terphenyldiyl group, a naphthalenediyl group, an anthracenediyl group, a phenanthrenediyl group, a pentalenediyl group, an indenediyl group, a heptalenediyl group, an indacenediyl group, a binaphthyldiyl group, a phenylnaphthalenediyl group, a stilbenediyl group, and a fluorenediyl group.
In the divalent heterocyclic group, the number of carbon atoms constituting its heterocyclic ring is generally about 3 to about 60, and specific examples may include a pyridinediyl group, a diazaphenylene group, a quinolinediyl group, a quinoxalinediyl group, an acridinediyl group, a bipyridinediyl group, a phenanthrolinediyl group, and a thiophenediyl group. The divalent heterocyclic group is preferably a divalent aromatic heterocyclic group.

When Q is an arylene group or a divalent heterocyclic group, a hydrogen atom contained in these groups may be substituted with a halogen atom, an alkyl group, an alkoxy group, an aryl group, or a monovalent heterocyclic group. The definitions and specific examples of the halogen atom, alkyl group, alkoxy group, aryl group, and monovalent heterocyclic group are the same as the definitions and specific examples of the halogen atom, alkyl group, alkoxy group, aryl group, and monovalent heterocyclic group represented by R described above.

Preferably, the fullerene derivatives of the present invention comprise two to four structures represented by the formula (1). More preferably, from the viewpoint of ease of synthesis, the fullerene derivatives comprise two structures represented by the formula (1).

Preferably, the fullerene derivatives of the present invention are fullerene derivatives represented by the formula (2) below.

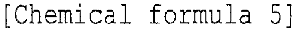

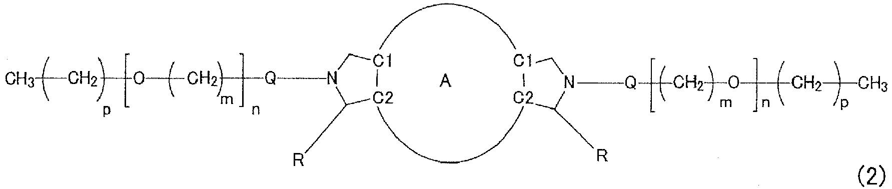

In the formula (2), an A ring represents a fullerene skeleton having 60 or more carbon atoms. The definitions of the C1, C2, m, n, p, Q, and R are the same as the definitions of those in the formula (1). Two Q's may be the same or different from each other. Two R's may be the same or different from each other. A plurality of m's may be the same or different from each other. Two n's may be the same or different from each other. Two p's may be the same or different from each other.

In the structure of the formula (1) and the fullerene derivatives of the formula (2), one preferred form of R is a group represented by the formula (3) below.

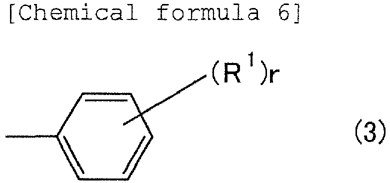

In the group of the formula (3), R¹ represents an alkyl group, an alkoxy group, an aryl group, a monovalent heterocyclic group, or a halogen group. r represents an integer from 0 to 5. When R¹ is plurally present, they may be the same or different from each other.

One preferred embodiment of the fullerene derivatives of the present invention may be the fullerene derivatives of the formula (2) in which the A ring is a fullerene skeleton having 60 or more carbon atoms, R is the group represented by the formula (3), R¹ is a methoxy group, m is 2, n is 2, p is 0, Q is a single bond, and r is 1.

In the fullerene derivatives of the formula (2), no particular limitation is imposed on the number of carbon atoms constituting the A ring. Even when any of various fullerenes having different numbers of carbon atoms such as a C₆₀ fullerene, a C₇₀ fullerene, a C₇₄ fullerene, a C₇₆ fullerene, a C₇₈ fullerene, and a C₈₄ fullerene is selected as the fullerene constituting the A ring, the effect of the invention described above can be obtained. From the viewpoint of availability of raw materials, the A ring is preferably a C₆₀ fullerene or a C₇₀ fullerene.

When the fullerene derivatives of the present invention are derivatives of the C₆₀ fullerene, specific examples thereof may include compounds (a) to (h) below.

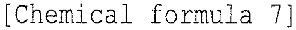

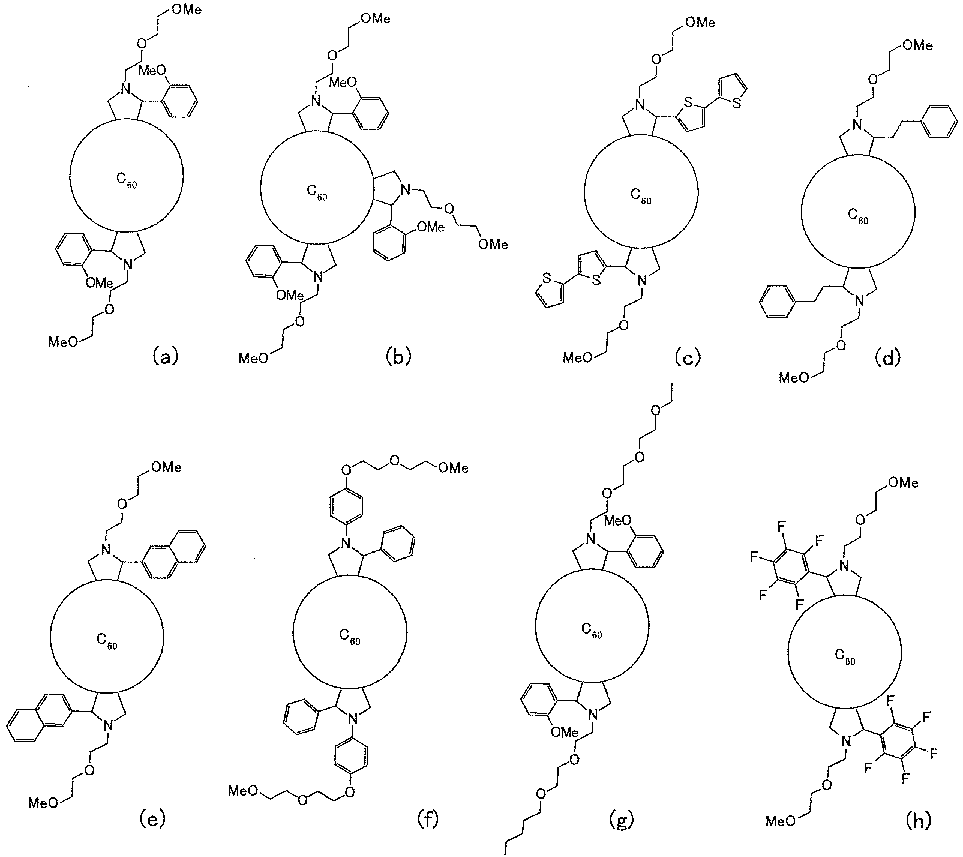

In the above fullerene derivatives, the rings denoted by C₆₀ are fullerene rings having 60 carbon atoms.

When the fullerene derivatives of the present invention are derivatives of the C₇₀ fullerene, specific examples thereof may include a compound (i) below.

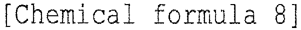

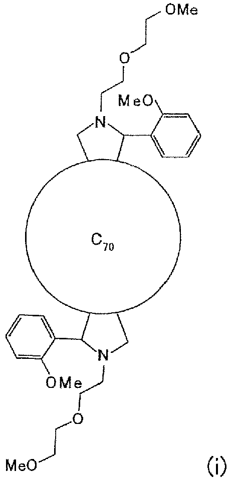

In the compound (i), the ring denoted by C₇₀ is a fullerene ring having 70 carbon atoms.
In the fullerene derivatives of the formula (2) and the compounds (a) to (i), no particular limitation is imposed on the positions of carbon atoms bonded to a plurality of structures (added groups) added to each fullerene ring, i.e., on the relative positional relation between the added groups.

The fullerene derivatives of the present invention can be synthesized (prepared) by, for example, a method known as a 1,3-dipolar cycloaddition reaction (Prato reaction) of a fullerene with iminium cations produced by decarbonation of an imine prepared from a glycine derivative and an aldehyde (see Accounts of Chemical Research Vol.31, 1998, p519-526).

Examples of the glycine derivative used in the above preparation method may include N-methoxymethylglycine and N-(2-(2-methoxyethoxy)ethyl)glycine.
The amount of the glycine derivative used is generally in the range of 0.1 moles to 10 moles based on 1 mole of the fullerene and preferably 0.5 moles to 3 moles.

Examples of the aldehyde which is the other raw material may include benzaldehyde and naphthaldehyde.
The amount of the aldehyde used is generally in the range of 0.1 moles to 10 moles based on 1 mole of the fullerene and preferably 0.5 moles to 4 moles.

The synthesis reaction of the fullerene derivatives of the present invention is generally performed in a solvent. A solvent inert to the synthesis reaction such as toluene, xylene, hexane, octane, or chlorobenzene is used as the above solvent. The amount of the solvent used is generally in the range of 1 to 100,000 times by weight the amount of the fullerene.

In the synthesis reaction, for example, the glycine derivative, aldehyde, and fullerene are mixed in a solvent, and the mixture is heated and reacted. The temperature of the synthesis reaction is generally in the range of 50°C to 350°C. The time of the synthesis reaction is generally 30 minutes to 50 hours.
After completion of the synthesis reaction under heating, the obtained reaction product is allowed to cool to room temperature, and the solvent is removed by evaporation under reduced pressure using a rotary evaporator. The obtained solids are separated and purified by silica gel flash column chromatography. The target fullerene derivative can be obtained by the above process.

The number of additional groups (structures) to be added to the fullerene can be controlled by appropriately adjusting the amounts of the glycine derivative and aldehyde used as the raw materials and the reaction conditions such as the reaction time and appropriately adjusting the separation-purification conditions, and a fullerene derivative having the desired number of additional groups added thereto can thereby be selectively obtained.

The fullerene derivatives of the present invention can be used as an electron acceptor compound and also as an electron donor compound. However, the fullerene derivatives are particularly preferably used as an electron acceptor compound. The fullerene derivatives of the present invention can be particularly preferably used as a material for an active layer formed by a coating method.
No particular limitation is imposed on the form of a composition comprising the fullerene derivatives of the present invention. For example, when the fullerene derivatives of the present invention are used for a coating composition used in a coating method, the fullerene derivatives are mixed with a suitable solvent to form a liquid such as a solution.

In a photovoltaic cell, when an active layer is formed as a layered structure in which a layer comprising an electron acceptor compound (an electron acceptor layer) is joined to a layer comprising an electron donor compound (an electron donor layer), the fullerene derivatives of the present invention can be used as a constituent component of the electron acceptor layer so as to serve as an electron acceptor compound.

In a photovoltaic cell, when the fullerene derivatives of the present invention is used for an active layer comprising both an electron acceptor compound and an electron donor compound, a composition comprising the fullerene derivatives of the present invention and the electron donor compound may be used.

It is preferable, from the viewpoint of coating properties, that the electron donor compound is a macromolecular compound. In the specification, the polystyrene equivalent number average molecular weight of the macromolecular compound is preferably 10³ or larger, and the polystyrene equivalent number average molecular weight may be generally 10⁸ or lower.
The macromolecular compound may be polyvinylcarbazole and derivatives thereof, polysilane and derivatives thereof, polysiloxane derivatives having an aromatic amine on their side chain or main chain, polyaniline and derivatives thereof, polythiophene and derivatives thereof, polypyrrole and derivatives thereof, polyphenylene vinylene and derivatives thereof, polythienylene vinylene and derivatives thereof, and polyfluorene and derivatives thereof.

It is preferable, from the viewpoint of photovoltaic efficiency, that the electron donor compound used for the organic photovoltaic cell is a macromolecular compound having a weight average molecular weight of about 5 x 10³ to about 10⁶ and comprising a repeating unit selected from the group consisting of repeating units of the formulae (4) and (5) below. More preferably, the electron donor compound is a macromolecular compound comprising the repeating unit represented by the formula (4) below.

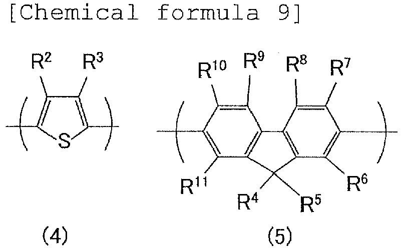

In the repeating units of the formulae (4) and (5), R², R³, R⁴, R⁵, R⁶, R⁷, R⁸, R⁹, R¹⁰, and R¹¹ may be the same or different from each other and each independently represent a hydrogen atom, an alkyl group, an alkoxy group, or an aryl group.

In the repeating unit of the formula (4), when R² and/or R³ is an alkyl group, specific examples thereof may include the alkyl groups exemplified for R described above. When R² and/or R³ is an alkoxy group, specific examples thereof may include the alkoxy groups exemplified for R described above. When R² and/or R³ is an aryl group, specific examples thereof may include the aryl groups exemplified for R described above.

In the repeating unit of the formula (4), it is preferable, from the viewpoint of photovoltaic efficiency, that at least one of R² and R³ be an alkyl group having 1 to 20 carbon atoms. More preferably, at least one of R² and R³ is an alkyl group having 4 to 8 carbon atoms.
Preferred examples of the macromolecular compound comprising the repeating unit of the formula (4) may include P3HT in which R² is H and R³ is C₆H₁₃.

In the repeating unit of the formula (5), when any of R⁴ to R¹¹, a combination thereof, or all of them are alkyl groups, specific examples thereof may include the alkyl groups exemplified for R described above. When any of R⁴ to R¹¹,a combination thereof, or all of them are alkoxy groups, specific examples thereof may include the alkoxy groups exemplified for R described above. When any of R⁴ to R¹¹, a combination thereof, or all of them are aryl groups, specific examples thereof may include the aryl groups exemplified for R described above.

In the repeating unit of the formula (5), it is preferable, from the viewpoint of ease of synthesis of the monomer, that R⁶ to R¹¹ be each a hydrogen atom. From the view point of photovoltaic efficiency, R⁴ and R⁵ are each preferably an alkyl group having 1 to 20 carbon atoms or an aryl group having 6 to 20 carbon atoms and more preferably an alkyl group having 5 to 8 carbon atoms or an aryl group having 6 to 15 carbon atoms.

When the active layer comprises the fullerene derivatives of the present invention and an electron acceptor, the amount of the fullerene derivative is preferably 10 parts by weight to 1,000 parts by weight based on 100 parts by weight of the electron donor compound and more preferably 50 parts by weight to 500 parts by weight.

### <Organic photovoltaic cell>

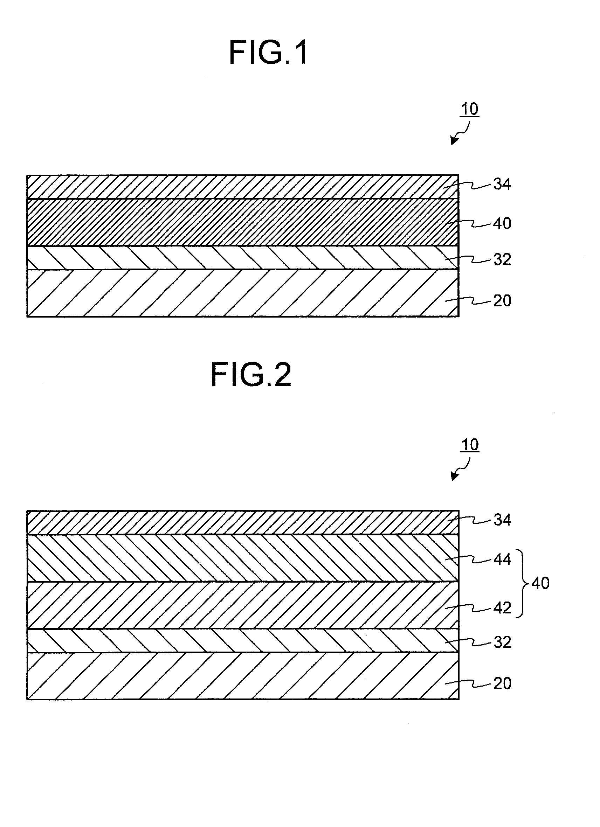

Constitution example of an organic photovoltaic cell of the present invention will be described with reference to FIGs. 1 and 2.
FIG. 1 is a schematic cross-sectional view illustrating a constitution example (1) of the organic photovoltaic cell. FIG. 2 is a schematic cross-sectional view illustrating a constitution example (2) of the organic photovoltaic cell.

The organic photovoltaic cell of the present invention comprises a pair of electrodes composed of an anode and a cathode and a layer placed between the pair of electrodes and comprising the fullerene derivative.

Among the pair of electrodes, at least an electrode on the side on which light goes, i.e., at least one of the electrodes, is a transparent or semi-transparent electrode that allows the incident light to pass therethrough.

### Constitution example (1)

As shown in FIG. 1, an organic photovoltaic cell 10 of the constitution example (1) comprises: a pair of electrodes comprising a first electrode 32 serving as, for example, an anode and a second electrode 34 serving as, for example, a cathode; and an active layer 40 placed between the pair of electrodes and comprising the fullerene derivative. The polarities of the first electrode 32 and the second electrode 34 may be appropriately set according to the structure of the cell. The first electrode 32 may be used as a cathode, and the second electrode 34 may be used as an anode.

Generally, an organic photovoltaic cell is formed on a substrate. More specifically, the organic photovoltaic cell 10 is provided on a main surface of a substrate 20.

Any material may be used for the substrate 20, so long as it is not chemically changed when the electrodes are formed and the layer comprising the organic material is formed. Examples of the material of the substrate 20 may include glass, plastic, polymer film, and silicon.

When the substrate 20 is non-transparent, the second electrode 34 opposed to the first electrode 32 and provided on the side opposite to the substrate (i.e., the electrode farther from the substrate 20) is preferably transparent or semi-transparent.

The active layer 40 is placed between the first electrode 32 and the second electrode 34 and in contact with these electrodes. The active layer 40 is, for example, an organic layer comprising an electron donor compound and the fullerene derivative of the present invention serving as an electron acceptor compound and is a layer having an essential function for the photovoltaic function.

The first electrode 32 is provided on the main surface of the substrate 20. The active layer 40 is provided so as to cover the first electrode 32. The second electrode 34 is provided in contact with the surface of the active layer 40.

### Constitution example (2)

As shown in FIG. 2, an organic photovoltaic cell in the constitution example (2) comprises: a pair of electrodes comprising an anode 32 and a cathode 34; and an active layer 40 placed between the pair of electrodes. The active layer 40 comprises: an electron acceptor layer 44 comprising the fullerene derivative of the present invention; and an electron donor layer 42 joined to the electron acceptor layer and comprising an electron donor compound.

The organic photovoltaic cell 10 is provided on the main surface of a substrate 20. The first electrode 32 is provided on the main surface of the substrate 20.

The active layer 40 is placed between the first electrode 32 and the second electrode 34 and in contact with these electrodes. The active layer 40 in the constitution example 2 has a layered structure in which, for example, the electron acceptor layer 44 comprising the fullerene derivative of the present invention as an electron acceptor compound is joined to the electron donor layer 42 comprising an electron donor compound.

The electron donor layer 42 is provided so as to join to the first electrode 32. The electron acceptor layer 44 is provided so as to join to the electron donor layer 32. The second electrode 34 is provided so as to join to the electron acceptor layer 44.

In the description of the constitution examples (1) and (2), the fullerene derivatives of the present invention are used as an electron acceptor compound. However, the fullerene derivatives of the present invention may be added, as an electron donor compound, to the active layer 40 in the constitution example (1) or to the electron donor layer 42 in the constitution example (2).

In the organic photovoltaic cell 10 of the constitution example (1), the active layer 40 has a configuration in which comprises the electron acceptor compound and the electron donor compound in a single layer. Therefore, the organic photovoltaic cell 10 comprises a larger area of hetero junction interface and is preferred from the viewpoint of improvement in photovoltaic efficiency.

The organic photovoltaic cell 10 may comprise an additional layer between at least one of the first electrode 32 and the second electrode 34 and the active layer comprising the fullerene derivatives of the present invention. Examples of the additional layer may include charge transport layers for transporting holes and electrons (a hole transport layer and an electron transport layer).

Any well-known appropriate material may be used for the materials constituting the charge transport layers. When the charge transport layer is an electron transport layer, examples of the material may include 2,9-dimethyl-4,7-diphenyl-1,10-phenanthroline (BCP). When the charge transport layer is a hole transport layer, examples of the material may include polyethylenedioxythiophene (PEDOT).

The additional layer optionally provided between the layer comprising the fullerene derivative and the first electrode 32 and/or the second electrode 34 may be a buffer layer. The material used for the buffer layer may be a halide of an alkali metal and halide of an alkaline-earth metal such as lithium fluoride; and an oxide such as titanium oxide. When an inorganic semiconductor is used, it may be used in a form of fine particles.

Examples of the possible layered structure of the organic photovoltaic cell in this embodiment are as follows:
a) anode / active layer / cathode;
b) anode / hole transport layer / active layer / cathode;
c) anode / active layer / electron transport layer /cathode;
d) anode / hole transport layer / active layer / electron transport layer / cathode;
e) anode / electron donor layer /electron acceptor layer /cathode;
f) anode / hole transport layer / electron donor layer /electron acceptor layer / cathode;
g) anode / electron donor layer / electron acceptor layer /electron transport layer / cathode; and
h) anode / hole transport layer / electron donor layer /electron acceptor layer / electron transport layer /cathode
(wherein the symbol "/" means that layers sandwiching the "/" are stacked adjacent to each other).

In the above layered structures, the anode may be provided on the side closer to the substrate, or the cathode may be provided on the side closer to the substrate.
In the organic photovoltaic cell 10 in the constitution example (1), the amount of the fullerene derivative in the active layer 40 comprising the electron donor compound and the fullerene derivative serving as an electron acceptor compound is preferably 10 parts by weight to 1,000 parts by weight based on 100 parts by weight of the electron donor compound and more preferably 50 parts by weight to 500 parts by weight.

In the organic photovoltaic cell 10 in the constitution example (1), the active layer 40 comprising the fullerene derivative and the electron donor compound can be formed using a composition comprising the fullerene derivative and the electron donor compound.

Preferably, a layer (the active layer 40, the electron donor layer 42, or the electron acceptor layer 44) comprising a fullerene derivative that can be used for the organic photovoltaic cells 10 is formed as an organic thin film comprising the fullerene derivative. The thickness of the organic thin film is generally 1 nm to 100 µm, preferably 2 nm to 1,000 nm, more preferably 5 nm to 500 nm, and still more preferably 20 nm to 200 nm.

When the first electrode 32 and/or the second electrode 34 is transparent or semi-transparent, examples of the material used may include conductive metal oxide films and semi-transparent metal thin films. More specifically, any of conductive material films of indium oxide, zinc oxide, tin oxide, and mixtures thereof such as indium tin oxide (ITO) and indium zinc oxide, NESA, and films of gold, platinum, silver, and copper can be used for the first electrode 32 and/or the second electrode 34. Among these, ITO, indium zinc oxide, and tin oxide are preferred. Examples of the method for forming the electrodes (the first electrode 32 and the second electrode 34) may include vacuum deposition method, sputtering method, ion plating method, and plating method.
Any of organic transparent conductive films of polyaniline and derivatives thereof and polythiophene and derivatives thereof may be used for the first electrode 32 and/or the second electrode 34.

Preferably, a material having a low work function is used as the electrode material of one of the first electrode 32 and the second electrode 34. The electrode comprising a material having a low work function may be transparent or semi-transparent. Examples of the material that can be used may include a metal such as lithium, sodium, potassium, rubidium, cesium, magnesium, calcium, strontium, barium, aluminum, scandium, vanadium, zinc, yttrium, indium, cerium, samarium, europium, terbium, and ytterbium; an alloy of two or more of the above metals; an alloy of at least one of the above metals and at least one metal selected from gold, silver, platinum, copper, manganese, titanium, cobalt, nickel, tungsten, and tin; graphite; and graphite intercalation compounds. Examples of the alloys may include magnesium-silver alloys, magnesium-indium alloys, magnesium-aluminum alloys, indium-silver alloys, lithium-aluminum alloys, lithium-magnesium alloys, lithium-indium alloys, and calcium-aluminum alloys.

The operation mechanism of the photovoltaic cell will next be described. The energy of light going through a transparent or semi-transparent electrode is absorbed by the electron acceptor compound and/or the electron donor compound, and thereby generating excitons in which electrons and holes are combined. When the generated excitons migrate and reach the hetero junction interface at which the electron acceptor compound and the electron donor compound are adjacent (joined) to each other, the electrons and the holes are separated from each other because of the differences between the HOMO and LUMO energies at the interface, and thereby generating charges (electrons and holes) that can move independently. The generated charges move toward the electrodes, and electric energy (electric current) can be extracted to out of the photovoltaic cell.

### <Method for forming organic thin films>

No particular limitation is imposed on the method for forming the organic thin films. Examples of the forming method may include a film forming method in which a solution comprising the fullerene derivatives used in the present invention is prepared and used.

No particular limitation is imposed on the solvent used for the solution, so long as the solvent can dissolve the fullerene derivatives of the present invention. Examples of the solvent may include: hydrocarbon solvents such as toluene, xylene, mesitylene, tetralin, decalin, bicyclohexyl, butylbenzene, sec-butylbenzene, and tert-butylbenzene; halogenated saturated hydrocarbon solvents such as carbon tetrachloride, chloroform, dichloromethane, dichloroethane, chlorobutane, bromobutane, chloropentane, bromopentane, chlorohexane, bromohexane, chlorocyclohexane, and bromocyclohexane; halogenated unsaturated hydrocarbon solvents such as chlorobenzene, dichlorobenzene, and trichlorobenzene; and ether-based solvents such as tetrahydrofuran and tetrahydropyran. The fullerene derivatives can be dissolved in any of the above solvents in an amount of generally 0.1 percent by weight or higher.

The solution may further comprise the above-described macromolecular compound. Specific examples of the solvent used for the solution may include the solvents exemplified above. However, from the viewpoint of the ability to dissolve the macromolecular compound, aromatic hydrocarbon solvents are preferred, and toluene, xylene, and mesitylene are more preferred.

Any of coating methods such as spin coating method, casting method, micro-gravure coating method, gravure coating method, bar coating method, roller coating method, wire bar coating method, dip coating method, spray coating method, screen printing method, flexography method, offset printing method, inkjet printing method, dispenser printing method, nozzle coating method, and capillary coating methods can be uses as the film forming method using the above-described solution. Among these, spin coating method, flexography method, inkjet printing method, and dispenser printing method are preferred.

When the organic photovoltaic cell is irradiated with light such as sunlight through the transparent or semi-transparent electrode, photovoltaic force is generated between the electrodes that sandwich the active layer, and the photovoltaic cell can thereby be operated as an organic thin film solar cell. By integrating a plurality of organic thin film solar cells, the integrated product can be used as an organic thin film solar cell module.

When the organic photovoltaic cell is irradiated with light through the transparent or semi-transparent electrode with a voltage being applied between the electrodes, a photocurrent flows. In this manner, the organic photovoltaic cell can be operated as an organic optical sensor. By integrating a plurality of organic optical sensors, the integrated product can be used as an organic image sensor.

### [Examples]

Examples are shown below to describe the present invention in more detail, but the invention is not limited to the Examples.

Commercial reagents and solvents were used for synthesis (formation) without any treatment or were used after distillation and purification in the presence of a desiccating agent. The C₆₀ fullerene used was a product of Frontier Carbon Corporation. NMR spectrum measurement was performed using MH500 by JEOL Ltd., and tetramethylsilane (TMS) was used as an internal standard. Infrared absorption spectrum measurement was performed using FT-IR 8000 by Shimadzu Corporation. MALDI-TOF MS spectrum measurement was performed using AutoFLEX-T2 by BRUKER.

### <Example 1> (Synthesis of fullerene derivative A)

### (Synthesis of benzyl [2-(2-hydroxyethoxy)ethylamino]acetate

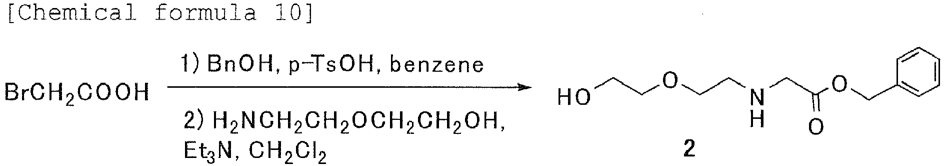

[First step] A two-neck flask equipped with a Dean-Stark trap was charged with bromoacetic acid (20.8 g, 150 mmol), benzyl alcohol (16.2 g, 150 mmol), para-toluenesulfonic acid (258 mg, 1.5 mmol), and benzene (300 mL), and the mixture was subjected to dehydration condensation at 120°C for 24 hours. The solvent was removed by evaporation under reduced pressure using an evaporator, and the resultant mixture was purified by silica gel flash column chromatography (hexane : ethyl acetate = 10:1, 5:1) to quantitatively obtain bromoacetic acid benzyl ester (34.3 g, 150 mmol) as a yellow oily product.

R_{f} 0.71 (hexane : ethyl acetate = 4:1);
¹H NMR (500 MHz, ppm, CDCl₃) δ 3.81 (s, 2H), 5.14 (s, 2H), 7.31 (s, 5H);
¹³C NMR (125 MHz, ppm, CDCl₃) δ 25.74, 67.79, 128.27, 128.48, 128.54, 134.88, 166.91;
IR (neat, cm⁻¹) 2959, 1751, 1458, 1412, 1377, 1167, 972, 750, 698.

[Second step] Triethylamine (17 mL, 120 mmol) was added to a dichloromethane (90 mL) solution of the bromoacetic acid benzyl ester (13.7 g, 60 mmol) at 0°C in an argon atmosphere, and the obtained solution mixture was stirred at 0°C for 20 minutes. Then a dichloromethane (40 mL) solution of 2-(2-aminoethoxy)ethanol (12 mL, 120 mmol) was added, and the resultant mixture was stirred at room temperature for 4 hours. Then the organic phase was washed three times with water and dried over anhydrous magnesium sulfate, and the solvent was removed by evaporation under reduced pressure using an evaporator. The resultant organic phase was purified by silica gel flash column chromatography (eluent: ethyl acetate : methanol = 1:0, 10:1, 5:1) to obtain glycine ester 2 (12.2 g, 48.0 mmol) as a colorless oily product. The yield was 80%.

R_{f} 0.48 (ethyl acetate : methanol = 2:1);
¹H NMR (500 MHz, ppm, CDCl₃) δ 2.83 (t, 2H, J = 5.1 Hz), 3.50 (s, 2H), 3.52 (t, 2H, J = 4.6 Hz), 3.58 (t, 2H, J = 5.0 Hz), 3.65 (t, 2H, J = 4.6 Hz), 5.11 (s, 2H), 7.28-7.30 (m, 5H) ;
¹³C NMR (125 MHz, ppm, CDCl₃) δ 48.46, 50.25, 61.29, 66.38, 69.80, 72.23, 126.63, 128.12, 128.37, 135.30, 171.78; IR (neat, cm⁻¹) 3412, 2880, 1719, 1638, 1560, 1508, 1458, 1067, 669.

### (Synthesis of [2-(2-methoxyethoxy)ethylamino]acetic acid)

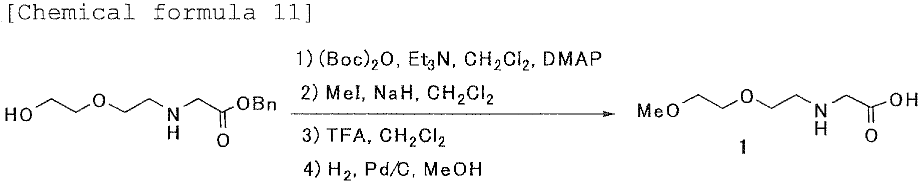

### [First step]

Triethylamine (4.3 mL, 31 mmol) was added to a dichloromethane (50 mL) solution of the benzyl[2-(2-hydroxyethoxy)ethylamino]acetate 2 (6.58 g, 26 mmol) at 0°C in an argon atmosphere. Then 4-(N,N-dimethylamino)pyridine (DMAP) (32 mg, 0.26 mmol) was added, and the obtained solution mixture was stirred for 20 minutes. A dichloromethane (10 mL) solution of di-tert-butyldicarbonate (6.77 g, 31 mmol) was added dropwise to the resultant solution mixture. Then the reaction mixture was stirred at room temperature for four hours and poured into an Erlenmeyer flask containing water to terminate the reaction. Then the reaction mixture was extracted three times with diethyl ether. The organic phase was dried, concentrated under reduced pressure, and then purified by silica gel flash column chromatography (eluent: hexane : ethyl acetate = 3:1, 2.5:1, 2:1) to obtain benzyl{tert-butoxycarbonyl-[2-(2-hydroxy-ethoxy)ethyl]amino}acetate 1 (5.83 g, 16.5 mmol) as a colorless oily product (5.83 g, 16.5 mmol). The yield was 63%.

R_{f} 0.58 (ethyl acetate : methanol = 20:1);
¹H NMR (500 MHz, ppm, CDCl₃) δ 1.34 (d, 9H, J = 54.5 Hz), 2.19 (brs, 1H), 3.38-3.45 (m, 4H), 3.50-3.60 (m, 4H), 3.99 (d, 2H, J = 41.3 Hz), 5.09 (d, 2H, J = 4.1 Hz), 7.25-7.30 (m, 5H);
¹³C NMR (125 MHz, ppm, CDCl₃) δ 27.82, 28.05, 47.90, 48.20, 49.81, 50.39, 61.23, 66.42, 69.92, 72.12, 80.08, 127.93, 128.14, 135.25, 154.99, 155.19, 169.94, 170.07;
IR (neat, cm⁻¹) 3449, 2934, 2872, 1751, 1701, 1458, 1400, 1367, 1252, 1143;
Anal. Calcd for C₁₈H₂₇NO₆: C, 61.17; H, 7.70; N, 3.96.
Found: C, 60.01; H, 7.75; N, 4.13.

### [Second step]

A tetrahydrofuran (THF) (20 mL) solution of the benzyl{tert-butoxycarbonyl-[2-(2-hydroxy-ethoxy)ethyl]amino}acetate (5.83 g, 16.5 mmol) was added dropwise to a THF (10 mL) solution of sodium hydride (1.2 g, 24.8 mmol, 50% in meneral oil) at 0°C in an argon gas atmosphere, and the mixture was stirred at 0°C for 20 minutes. Then iodomethane (1.6 mL, 24.8 mmol) was added to the mixture at 0°C. The reaction mixture was stirred at room temperature for 20 hours, and water was added thereto while the mixture was cooled in an ice bath to terminate the reaction. The mixture was extracted three times with ether, and the organic phase was dried, concentrated under reduced pressure, and purified by silica gel flash column chromatography (eluent: hexane : ethyl acetate = 5:1, 3:1) to obtain benzyl{tert-butoxycarbonyl-[2-(2-methoxy-ethoxy)ethyl]amino}acetate (3.02 g, 8.21 mmol) as a colorless oily produced. The yield was 50%.

R_{f} 0.54 (hexane : ethyl acetate = 1:1);
¹H NMR (500 MHz, ppm, CDCl₃) δ 1.34 (d, 9H, J = 51.8 Hz), 3.28 (d, 3H, J = 2.7 Hz), 3.37-3.46 (m, 6H), 3.52 (dt, 2H,
J = 5.4Hz, 16.5 Hz), 4.02 (d, 2H, J = 34.8 Hz), 5.09 (d, 2H, J = 4.5 Hz), 7.24-7.30 (m, 5H);
¹³C NMR (125 MHz, ppm, CDCl₃) δ 24.93, 25.16, 44.68, 45.00, 46.70, 47.40, 55.78, 63.30, 67.22, 68.60, 76.95, 124.98, 125.14, 125.36, 132.49, 151.99, 152.31, 166.84, 166.96;
IR (neat, cm⁻¹) 2880, 1751, 1701, 1560, 1458, 1400, 1366, 1117, 698, 617;
Anal. Calcd for C₁₉H₂₉NO₆: C, 62.11; H, 7.96; N, 3.81.
Found: C, 62.15; H, 8.16; N, 3.83.

### [Third step]

Trifluoroacetic acid (TFA) (9.0 mL) was added to a dichloromethane (17 mL) solution of the benzyl{tert-butoxycarbonyl-[2-(2-methoxy-ethoxy)ethyl]amino}acetate (3.02 g, 8.21 mmol) in an argon atmosphere, and the mixture was stirred at room temperature for 7 hours. Then a 10% aqueous sodium carbonate solution was added to adjust the pH of the mixture to 10, and the resultant mixture was extracted with dichloromethane. The organic phase was dried over anhydrous magnesium sulfate and concentrated under reduced pressure to quantitatively obtain benzyl[2-(2-methoxy-ethoxy)ethylamino]acetate (2.19 g, 8.19 mmol) as a yellow oily product.

R_{f} 0.32 (ethyl acetate : methanol = 20:1);
¹H NMR (500 MHz, ppm, CDCl₃) δ 1.99 (brs, 1H), 2.83 (t, 2H, J = 5.3 Hz), 3.38 (s, 3H), 3.50 (s, 2H), 3.54 (t, 2H, J = 4.6 Hz), 3.60-3.62 (m, 4H), 5.17 (s, 2H), 7.32-7.38 (m, 5H);
¹³C NMR (125 MHz, ppm, CDCl₃) δ 48.46, 50.66, 58.76, 66.20, 70.00, 70.44, 71.64, 128.09, 128.33, 135.44, 171.84;
IR (neat, cm⁻¹) 3350, 2876, 1736, 1560, 1458, 1117, 1030, 698, 619;
Anal. Calcd for C₁₄H₂₁NO₄: C, 62.90; H, 7.92; N, 5.24. Found: C, 62.28; H, 8.20; N, 5.05.

### [Fourth step]

Activated carbon having 10 percent by weight of palladium supported thereon (Pd/C: 219 mg) was added to a methanol (27 mL) solution of the benzyl[2-(2-methoxy-ethoxy)ethylamino]acetate (2.19 g, 8.19 mmol) at room temperature, and the atmosphere was purged with hydrogen gas. Then the mixture was stirred at room temperature for 7 hours in a hydrogen atmosphere. The Pd/C was removed using a glass filter over which Celite had been spread, and the Celite layer was washed with methanol. The filtrate was concentrated under reduced pressure to obtain [2-(2-methoxyethoxy)ethylamino]acetic acid 1 (1.38 g, 7.78 mmol) as a yellow oily product. The yield was 95%.

¹H NMR (500 MHz, ppm, MeOD) δ 3.21 (t, 2H, J = 5.1 Hz), 3.38 (s, 3H), 3.51 (s, 2H), 3.57 (t, 2H, J = 4.4 Hz), 3.65 (t, 2H, J = 4.6 Hz), 3.73 (t, 2H, J = 5.1 Hz);
¹³C NMR (125 MHz, ppm, MeOD) δ 48.13, 50.49, 59.16, 67.08, 71.05, 72.85, 171.10;
IR (neat, cm⁻¹) 3414, 2827, 1751, 1630, 1369, 1111, 1028, 851, 799;
Anal. Calcd for C₇H₁₅NO₄: C, 47.45; H, 8.53; N, 7.90. Found: C, 46.20; H, 8.49; N, 7.43.

### (Synthesis of fullerene derivative A)

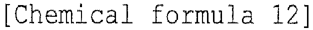

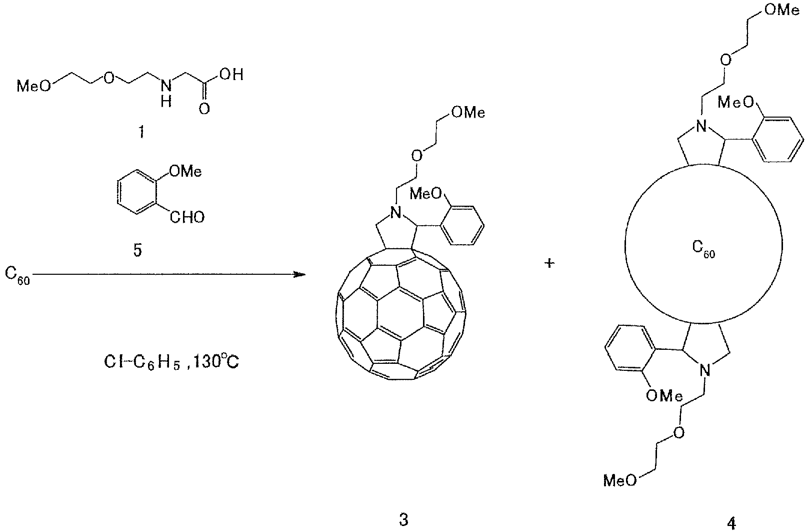

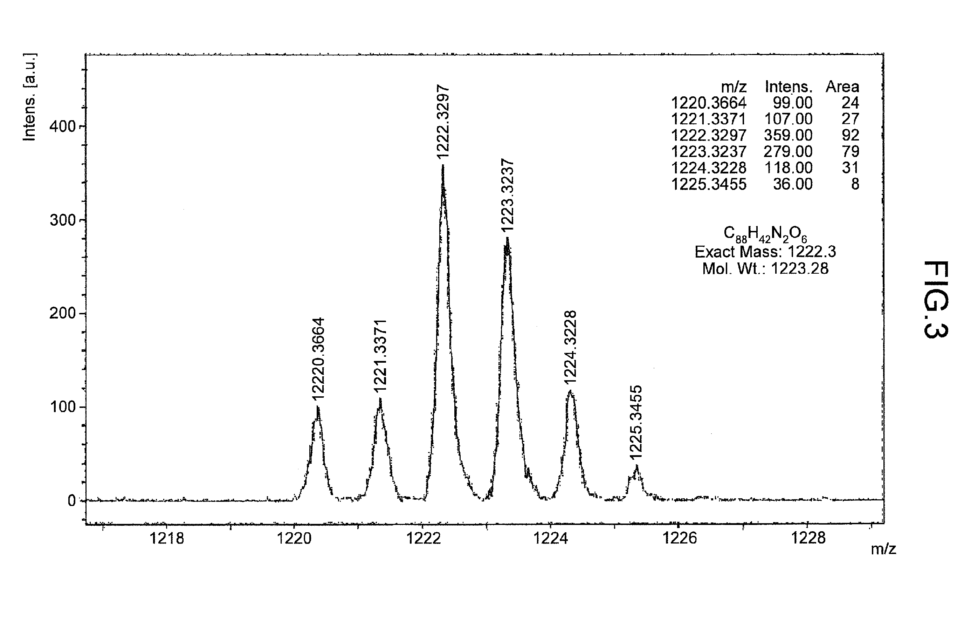

A two-neck flask (200 mL) equipped with a Dimroth condenser was charged with a C₆₀ fullerene (500 mg, 0.69 mmol), the glycine derivative 1(369 mg, 2.08 mmol), and 2-methoxybenzaldehyde 5 (470 mg, 3.45 mmol). Then chlorobenzene (100 mL) was added thereto, and the mixture was heated and refluxed for 3 hours. The resultant mixture was allowed to cool to room temperature, and the solvent was removed using a rotary evaporator. The resultant mixture was purified by silica gel flash column chromatography (toluene : ethyl acetate = 1:0 to 10:0 (volume ratio)) and then subjected to preparative thin-layer chromatography (carbon disulfide : ethyl acetate = 10:1 (volume ratio)) to obtain 95 mg (0.13 mmol) of a C₆₀ fullerene, 302 mg (0.31 mmol) of a mono adduct 3 (a fullerene derivative B), and 237 mg (0.19 mmol, yield 28%) of a di-adduct 4 (a fullerene derivative A) as a brown powder. FIG. 3 shows the MALDI-TOF MS spectrum of the fullerene derivative A.

### Fullerene derivative A:

IR (Neat, cm⁻¹) 2864, 2827, 1489, 1460, 1425, 1284, 1244, 1179, 1109, 1048, 1026, 754, 729, 527;
MALDI-TOF-MS (matrix: SA) found 1222.3297 (calcd for C₈₈H₄₂N₂O₆: Exact Mass: 1222.3, Mol. Wt.: 1223.28. m/e: 1222.30 (100.0%), 1223.31 (95.9%), 1224.31 (46.7%), 1225.31 (15.4%), 1226.32 (3.8%).

### Fullerene derivative B:

¹H NMR (500 MHz, ppm, CDCl₃, J = Hz) δ 2.77-2.82 (1H, m), 3.39 (3H, s), 3.61 (2H, t, J = 4.5 Hz), 3.69 (3H, s), 3.71-3.78 (2H, m), 3.92-4.02 (2H, m), 4.27 (1H, d, J = 9.6 Hz), 5.18 (1H, d, J = 9.6 Hz), 5.73 (1H, s), 6.85 (1H, d, J = 8.2 Hz), 7.01 (2H, t, J = 7.5 Hz), 7.20 (1H, m), 7.94 (1H, d, J = 7.8Hz); ¹³C NMR (125 MHz, ppm, CDCl₃) δ 52.19, 54.71, 58.77, 67.45, 69.17, 70.49, 70.57, 71.96, 73.92, 75.24, 76.75, 110.67, 121.10, 125.42, 128.80, 129.74, 134.32, 135.79, 136.21, 136.31, 139.09, 139.17, 139.86, 139.96, 141.27, 141.42, 141.52, 141.69, 141.78, 141.88, 141.96, 142.00, 142.05, 142.27, 142.33, 142.36, 142.71, 142.76, 144.08, 144.13, 144.29, 144.77, 144.94, 144.97, 145.03, 145.28, 145.44, 145.59, 145.74, 145.81, 145.86, 145.90, 145.94, 146.25, 146.47, 146.96, 153.86, 153.91, 154.76, 156.72, 157.79;
IR (Neat, cm⁻¹) 2864, 2827, 1489, 1460, 1425, 1284, 1244, 1179, 1109, 1048, 1026, 754, 729, 527; MALDI-TOF-MS (matrix: SA) found 971.1526 (calcd for C₇₄H₂₁NO₃ Exact Mass: 971.1521).

### <Example 2> (Preparation of composition and organic thin film and measurement of absorbance)

Regioregular P3HT (purchased from Sigma Aldrich, Mn ≅ 64,000) was dissolved in chlorobenzene at a concentration of 1% (percent by weight). The fullerene derivative A serving as an electron acceptor was mixed in an amount equal to the weight of the P3HT to prepare a solution. Next, the prepared solution was filtrated through a Teflon (registered trademark) filter with a pore diameter of 0.2 µm to prepare a coating solution. A glass substrate was spin-coated with the obtained coating solution. The coating operation was performed at 23°C. Then the substrate was baked under the conditions of 130°C in a nitrogen atmosphere for 10 minutes to obtain an organic thin film having a thickness of about 100 nm. The absorption spectrum of the organic thin film was measured using a spectrophotometer (product name: V-670, a product of JASCO Corporation). The absorbance at a wavelength of 600 nm is shown in Table 1.

### <Comparative Example 1>

An organic thin film was formed by the same procedure as in Example 2 except that [60]-PCBM (Phenyl C61-butyric acid methyl ester, product name: ADS61BFB, a product of American Dye Source Inc.) instead of the fullerene derivative A was mixed with the solution in an amount of 100 percent by weight based on the weight of the P3HT, and the absorption spectrum of the organic thin film was measured. The absorbance at a wavelength of 600 nm is shown in Table 1.

### <Comparative Example 2>

An organic thin film was produced by the same procedure as in Example 2 except that the fullerene derivative B instead of the fullerene derivative A was mixed with the solution in an amount of 100 percent by weigh based on the weight of the P3HT, and the absorption spectrum of the organic thin film was measured. The absorbance at a wavelength of 600 nm is shown in Table 1.

**[Table 1]**

| | FULLERENE DERIVATIVE | ABSORBANCE AT 600 nm |
|---|---|---|
| EXAMPLE 2 | FULLERENE DERIVATIVE A | 0.27 |
| COMPARATIVE EXAMPLE 1 | [60]-PCBM | 0.21 |
| COMPARATIVE EXAMPLE 2 | FULLERENE DERIVATIVE B | 0.23 |

As is clear from Table 1, the absorbance at a wavelength of 600 nm of the organic thin film of Example 2 comprising the fullerene derivative of the present invention was higher than those of the organic thin films of Comparative Examples 1 and 2. Therefore, the use of the fullerene derivative A of the present invention allows the light absorbance at a wavelength of 600 nm to be significantly improved.

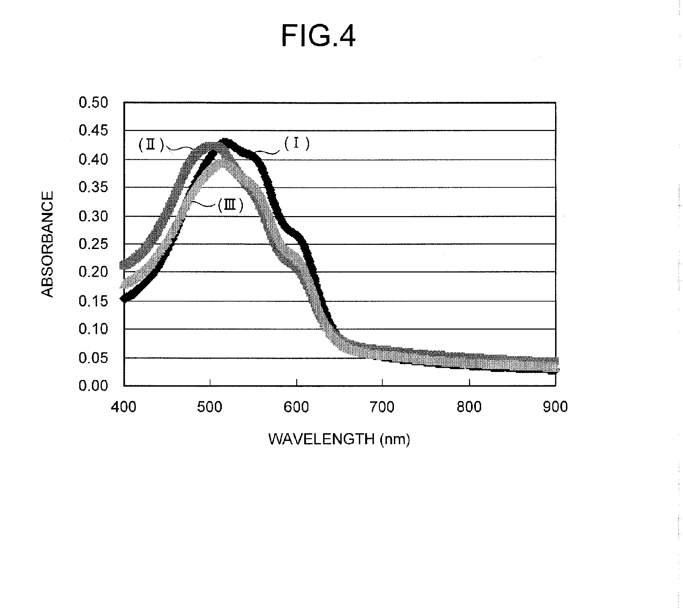

Referring to FIG. 4, the absorption spectrum of the organic thin films comprising the fullerene derivatives of Example 1 and Comparative Examples 1 and 2 will be further described.
FIG. 4 is a graph showing the absorption spectrum of the organic thin films each comprising a fullerene derivative and P3HT. The vertical axis represents absorbance (no unit), and the horizontal axis represents wavelength (nm). Graph (I) represents the absorption spectrum of the organic thin film comprising P3HT and the fullerene derivative of Example 1. Graph (II) represents the absorption spectrum of the organic thin film comprising P3HT and the fullerene derivative in Comparative Example 1. Graph (III) represents the absorption spectrum of the organic thin film comprising P3HT and the fullerene derivative of Comparative Example 2.

As is clear from FIG. 4, the organic thin film of the present invention that comprises the fullerene derivative of the present invention and further the electron donor compound (P3HT) has improved light absorbance in a long wavelength range, particularly in the wavelength range of about 500 nm to about 650 nm), as compared to the organic thin films each comprising P3HT and the fullerene derivative of Comparative Example 1 or 2.

When the fullerene derivatives of the present invention are used for organic photovoltaic cells, the cells can have improved light absorbance in the long wavelength range, particularly in the wavelength range of about 500 nm to about 650 nm, as compared to organic photovoltaic cells in which any of the fullerene derivatives of Comparative Examples 1 and 2 that correspond to conventional art is used.

### <Example 3> (Formation of organic thin film solar cell and validation of its operation)

Regioregular P3HT (a product of Aldrich, Lot#: 09007KH) serving as an electron donor compound was dissolved in chlorobenzene at a concentration of 1% (percent by weight). Then the fullerene derivative A serving as an electron acceptor compound was mixed in an amount equal to the weight of the electron donor compound to prepare a solution. Then 1 part by weight of silica gel (Wakogel C-300, a product of Wako Pure Chemical Industries, Ltd., particle diameter: 45 µm to 75 µm) used as an adsorbent was added to 100 parts by weight of the solution, and the mixture was stirred for 12 hours. The resultant mixture was filtrated through a Teflon (registered trademark) filter with a pore diameter of 1.0 µm to prepare a coating solution.

A glass substrate (substrate) having a 150 nm-thick ITO film (first electrode) formed thereon by sputtering method was subjected to ozone UV treatment as surface treatment. Next, the ITO film formed on the glass substrate was spin-coated with the coating solution to obtain an active layer (thickness: about 100 nm) of a photovoltaic cell (an organic thin film solar cell). Then the product was baked under the conditions of 130°C in a nitrogen atmosphere for 10 minutes. Then lithium fluoride was first deposited to a thickness of 4 nm using a vacuum deposition apparatus, and then Al was deposited to a thickness of 100 nm (a second electrode). The degree of vacuum during all the deposition processes was 1 x 10⁻³ Pa to 9 x 10⁻³ Pa. The shape of the obtained organic thin film solar cell was 2 mm square. The validation of the operation of the obtained organic thin film solar cell was performed by irradiating the organic thin film solar cell with light at a constant illuminance with an irradiance of 100 mW/cm² using a solar simulator (product name: OTENTO-SUNII, AM1.5G filter, a product of BUNKOUKEIKI Co., Ltd.). As a result. the organic thin film solar cell was found to operate properly.

### INDUSTRIAL APPLICABILITY

The fullerene derivatives of the present invention can be used for organic photovoltaic cells having high photovoltaic efficiency.

## Claims

1. A fullerene derivative comprising two or more structures represented by the following formula (1): 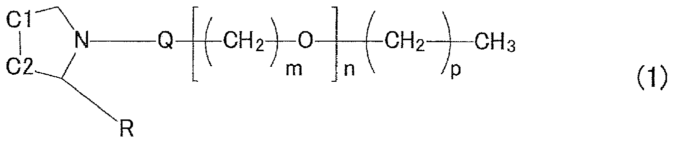 wherein, in the formula (1), C1 and C2 represent carbon atoms constituting a fullerene skeleton, m represents an integer from 1 to 6, n represents an integer from 1 to 4, p represents an integer 0 or more, R represents a monovalent organic group, and Q represents a single bond, an arylene group, or a divalent heterocyclic group, and wherein when m is plurally present, these m may be the same or different from each other.

2. The fullerene derivative according to claim 1, represented by the following formula (2): 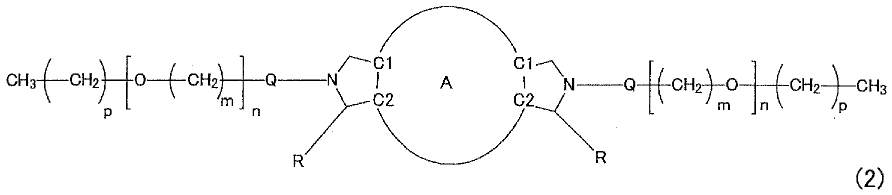 wherein, in the formula (2), an A ring represents a fullerene skeleton having 60 or more carbon atoms, and the definitions of C1, C2, m, n, p, Q, and R are the same as the definitions for the formula (1), and wherein two of Q may be the same or different from each other, two of R may be the same or different from each other, a plurality of m may be the same or different from each other, two of n may be the same or different from each other, and two of p may be the same or different from each other.

3. The fullerene derivative according to claim 1, wherein R is a group represented by the following formula (3): 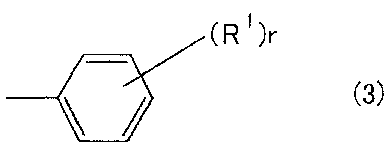 wherein, in the formula (3), R¹ represents an alkyl group, an alkoxy group, an aryl group, a monovalent heterocyclic group, or a halogen group, and r represents an integer from 0 to 5, and wherein when R¹ is plurally present, these R¹ may be the same or different from each other.

4. The fullerene derivative according to claim 3, wherein the A ring is a fullerene skeleton having 60 or more carbon atoms, R¹ is a methoxy group, m is 2, n is 2, p is 0, Q is a single bond, and r is 1.

5. A composition comprising the fullerene derivative according to claim 1 and an electron donor compound.

6. The composition according to claim 5, wherein the electron donor compound is a macromolecular compound.

7. An organic photovoltaic cell comprising:
a pair of electrodes comprising an anode and a cathode; and
an active layer placed between the pair of electrodes and comprising the fullerene derivative according to claim 1.

8. An organic photovoltaic cell comprising:
a pair of electrodes comprising an anode and a cathode; and
an active layer placed between the pair of electrodes and comprising the composition according to claim 5.

9. An organic photovoltaic cell comprising:
a pair of electrodes comprising an anode and a cathode; and
an active layer placed between the pair of electrodes, the active layer comprising an electron acceptor layer comprising the fullerene derivative according to claim 1 and an electron donor layer comprising an electron donor compound, the electron donor layer being joined to the electron donor layer.
